# NEW EUROPEAN PATENT SPECIFICATION

(11) **EP 2 938 199 B2**
(45) Date of publication and mention of the opposition decision: **02.11.2022**
(45) Mention of the grant of the patent: 03.07.2019
(21) Application number: 13818522.8
(22) Date of filing: 24.12.2013
(51) Int. Cl.: A23C 9/123, A23C 1/12, C12N 1/20

(54) **METHOD FOR PREPARING A CONCENTRATED FERMENTED DAIRY BASE**
VERFAHREN ZUR HERSTELLUNG EINES KONZENTRIERTEN FERMENTIERTEN MOLKEREIPRODUKTES
PROCÉDÉ DE PRÉPARATION D'UNE BASE LAITIÈRE FERMENTÉE CONCENTRÉE

(30) Priority: 28.12.2012 NL 2010069; 28.12.2012 US 201261746680 P; 28.01.2013 US 201361757313 P
(43) Date of publication of application: 04.11.2015
(73) Proprietor: DSM IP Assets B.V., 6411 TE Heerlen (NL)
(72) Inventor: HOLTMEYER, André, NL-7623 PM Borne (NL); SMALBRINK, Lex, NL-6712 EL Ede (NL); MEIJER, Willem Cornelis, 6721 AM Bennekom (NL); HAFKAMP, Albertus Antonius Gerardus, NL-6974 AV Leuvenheim (NL)
(74) Representative: DSM Intellectual Property
(86) International application number: PCT/NL2013/050954
(87) International publication number: WO 2014/104887

(56) References cited:
- WO-A1-03/071883
- US-A- 5 656 268
- US-A- 6 156 353
- US-A1- 2009 304 864

## Description

### Field of the invention

The present invention is in the field of fermented milk-based compositions, and in particular relates to yoghurt powder.

### Background of the invention

The present invention relates to a method for preparing a fermented dairy base. The invention further provides a frozen starter culture.

Fermented milk powders, especially yoghurt-type powders, are widely used in industry. Commercially relevant applications of these powders are in the manufacture of fillings, toppings or desserts or as ingredient in dried rehydratable foodstuffs. Fermented milk powders are typically produced in a process involving drying a fermented dairy base. Such fermented dairy bases are commonly obtained by culturing a dairy base in the presence of a starter comprising a strain of *Streptococcus thermophilus* and a strain of *Lactobacillus delbrueckii* subsp. *bulgaricus.* At the start of the culturing process, the dairy base typically has a pH of between 6-7. The culturing process is typically completed at a pH of about 4.7. For some applications a pH of 4.5 or lower is required in order to obtain a desired enhanced acidity. A fermented milk powder is conveniently manufactured in a process involving (spray) drying the fermented dairy base.

The dairy base can be selected as milk *per se,* for example as cow milk. The content of non-fat dry milk solids in cow milk typically resides between 9-10%. In order to allow the drying process to take place as efficiently as possible, the dairy base is usually provided as a concentrated milk.

US 6,156,353 discloses fermentation in the presence of *Streptococcus thermophilus* and *Lactobacillus helveticus* of whole cow's milk to which corn oil, sucrose, maltodextrin and starch are added. The solution that is fermented has a milk solids non fat content of about 13%.

Example 1 of US 2009/304864 discloses a method to produce a fermented dairy base in the form of a yoghurt. This yoghurt is subsequently dried to produce a yoghurt powder. The method comprises preparing a dairy base ("dairy mixture") by incorporating skimmed milk powder into milk with 0% fat to obtain a dry extract of 20%, and inoculating this composition with a strain of *Streptococcus thermophilus* and a strain of *Lactobacillus delbrueckii* subsp. *bulgaricus.*

US 5,656,268 discloses a biological product comprising ferments of *Lactobacillus bulgaricum, Lactobacillus acidophilus, Lactobacillus helveticus, Lactobacillus casei* and *Streptococcus thermophilus* in dry form for various applications such as in cosmetic cream, massage oil, cream for gum treatment and as food additive for improving user's health.

### Summary of the invention

In order to further improve the efficiency of a process comprising drying of a fermented dairy base, it would be desirable to further increase the milk solids non fat content of the dairy base from 20% up to higher levels. However, it has been observed that using conventional starter cultures for yoghurt-type fermented dairy bases comprising a strain of *Streptococcus thermophilus* and a strain of *Lactobacillus delbrueckii* subsp. *bulgaricus,* the acidification rate of the dairy base during fermentation may suddenly decrease if the milk solids non fat content of the dairy based is raised from 20% upwards even by a few percent points.

More in particular, it was found that whilst a conventional starter culture comprising a strain of *Streptococcus thermophilus* and a strain of *Lactobacillus delbrueckii* subsp. *bulgaricus* could under commercially relevant conditions reduce the initial pH of a concentrated skim milk having a milk solids non fat content of 15-20% from a value of about 6.5 down to a value of about 4.7 within a period of 12 hours or less, the same starter culture when dosed in the same amount under the same conditions would be incapable to reduce the initial pH of a concentrated skim milk having a milk solids non fat content of 23% or higher from a value of about 6.5 down to a value of about 4.7 within 12 hours.

In an attempt to solve this problem, the present inventors surprisingly found that the incorporation of an additional strain of *Lactobacillus helveticus* in the starter surprisingly improved the acidification properties thereof, to the extent that the initial pH of the dairy base could be reduced from a value of about 6.5 down to a value of less than 4.7 during culturing within a period of 12 hours or less, even without having to enhance the total dosage of lactic acid bacteria to the dairy base prior to culturing. Also under conditions where practically no acidification by a starter culture comprising a strain of *Streptococcus thermophilus* and a strain of *Lactobacillus delbrueckii* subsp. *bulgaricus* took place, it was found that the incorporation of an additional strain of *Lactobacillus helveticus* in the starter surprisingly did result in acidifcation and even at a commercially interesting rate. Thus, it was surpsingly found, at least, that the additional strain of *Lactobacillus helveticus* in the starter advantageously improved the acidification properties thereof to a significant extent, even without having to enhance the total dosage of lactic acid bacteria to the dairy base prior to culturing.

Thus, the invention provides in a first aspect a method for producing a fermented dairy base comprising culturing a composition comprising milk, which comprises a milk solids non fat content of at least 23%, [[-]] in the presence of a starter culture comprising a strain of *Streptococcus thermophilus,* a strain of *Lactobacillus delbrueckii* subsp. *bulgaricus* and a strain of *Lactobacillus helveticus,* wherein prior to culturing, the composition comprising milk is inoculated with the starter culture to provide an initial
viable cell count of *Lactobacillus helveticus* of at least 5.10⁴ cfu per ml of composition comprising milk, of *Streptococus thermophilus* of at least 1.10⁴ cfu per ml of composition comprising milk and of *Lactobacillus delbrueckii* subsp. *Bulgaricus* of at least 1.10³ cfu per ml of composition comprising milk and wherein the starter culture is provided as a frozen starter culture and the frozen starter culture comprises
a. bacteria of *Streptococcus thermophilus* at a total viable cell count density of at least 1.10⁸ cfu/g of frozen starter culture, preferably of at least 1.10⁹ cfu/g;
b. bacteria of *Lactobacillus delbrueckii* subsp. *bulgaricus* at a total viable cell count density of at least 1.10⁷ cfu/g of frozen starter culture, preferably at least 1.10⁸ cfu/g; and
c. bacteria of *Lactobacillus helveticus* at a total viable cell count density of least 1.10⁸ cfu/g of frozen starter culture, preferably of at least 1.10⁹ cfu/g,
**and wherein the frozen starter culture comprises less than 1.10⁶ cfu/g Lactobacillus casei.**

The fermented dairy base thus obtained preferably has a liquid or semi-solid consistency so that it is processable into a powder preferably using spray drying equipment.

There is also described a fermented dairy base obtainable by the method for producing a fermented dairy base according to the present invention.

There is also provided a method for producing a powder comprising drying the fermented dairy base obtained according to the method for producing a fermented dairy base according to the present invention, and a powderobtainable by drying the fermented dairy base obtained according to the method for producing a fermented dairy base.

The invention also provides a frozen starter culture for preparing a fermented dairy base, the starter culture comprising
a. bacteria of *Streptococcus thermophilus* at a total viable cell count density of at least 1.10⁸ cfu/g of frozen starter culture, preferably of at least 1.10⁹ cfu/g;
b. bacteria of *Lactobacillus delbrueckii* subsp. *bulgaricus* at a total viable cell count density of at least 1.10⁷ cfu/g of frozen starter culture, preferably at least 1.10⁸ cfu/g; and
c. bacteria of *Lactobacillus helveticus* at a total viable cell count density of least 1.10⁸ cfu/g of frozen starter culture, preferably of at least 1.10⁹ cfu/g,
**and wherein the frozen starter culture comprises less than 1.10⁶ cfu/g Lactobacillus casei.**

The expression "cfu" stands for "colony forming units".

### Detailed description of the invention

### Definitions and conventions

The term "milk" is known to the skilled person and is preferably defined as the lacteal secretion obtained by the complete milking of a mammalian animal. Herein the mammalian animal is preferably selected from the group consisting of a sheep, a goat, a camel, a cow or a buffalo. The term "milk" may thus refer to sheep milk, goat milk, camel milk, cow milk, buffalo milk or a mixture thereof. In a preferred embodiment of this invention the milk comprises cow milk.

The expression "non-fat dry milk solids content" or equivalently "milk solids non fat content" or equivalently "content of milk solids non fat" is known to the skilled person and preferably relates to the total content of the proteins, carbohydrates, watersoluble vitamins and minerals contained by the milk. The content of non-fat dry milk solids of a milk can be suitably obtained as the total milk solids content of the milk minus its content of milk fat.

Unless otherwise indicated, percentages herein relate to weight/weight percentages or % (w/w). In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the elements is present, unless the context clearly requires that there be one and only one of the elements. The indefinite article "a" or "an" thus usually means "at least one".

The expression E9, E10 etc. means 10⁹, 10¹⁰ etc.

The determination of viable cell count density of the lactic acid bacterial strains described or claimed herein is known to the skilled person. The viable cell count density (expressed in cfu/g or in cfu/ml) of *Streptococcus thermophilus* is preferably determined using Streptococcus Thermophilus Isolation Agar or STA. This agar has the following composition: Casein enzymic hydrolysate 10.0 g/l; Yeast extract 5.0 g/l; Sucrose 10.0 g/l; Dipotassium phosphate 2.0 g/l; Agar 15.0 g/l; Final pH 6.8 +/- 0.2 at 25°C. Cultural characteristics after anaerobic incubation for 48-72 hours at 35-37°C. STA is conventiently obtained as Fluka cat.# 17257 Streptococcus Thermophilus Isolation Agar from Sigma-Aldrich Chemie GmbH, Industriestrasse 25, Postfach CH-9471 Buchs, Switzerland. In a mixed culture or in a frozen pellet comprising a mixture of Streptococcus thermophilus and lactobacilli, streptococci and lactobacilli can be together enumerated on the same STA plate; streptococci are counted as smooth, round colonies and lactobacilli are counted as fluffy colonies. In a single strain culture or in a frozen pellet comprising a single strain, the viable cell count density (expressed in cfu/g or in cfu/ml) of lactobacilli, especially of *Lactobacillus helveticus* is preferably determined using tryptone glucose meat extract agar for example as detailed by Galesloot, T. E., F. Hassing, and J. Stadhouders. 1961. Neth. Milk Dairy J. 15:127-150. Cultural characteristics are conveniently identifiable after incubation for 2-3 days at 37°C.

The phrase 'majority' as used herein means more than 50%.

### Further embodiments of the method according to the invention

Prior to culturing, the composition comprising milk is preferably inoculated with the starter culture to provide (1) an initial viable cell count of *Streptococus thermophilus* of at least 1.10⁴ cfu per ml of composition comprising milk, more preferably of 5.10⁴-1.10⁸ cfu per ml of composition comprising milk, or (2) an initial viable cell count of *Lactobacillus helveticus* of at least 5.10⁴ cfu per ml of composition comprising milk, preferably of between 5.10⁴-1.10⁹ cfu per ml of composition comprising milk, or (3) an initial viable cell count of *Lactobacillus delbrueckii* subsp. *bulgaricus* of at least 1.10³ cfu per ml of composition comprising milk, preferably 1.10⁴-1.10⁸ cfu per ml of composition comprising milk. In one embodiment it is preferred that prior to culturing, the composition comprising milk is inoculated with the starter culture to provide (1) an initial viable cell count of *Streptococus thermophilus* of at least 1.10⁴ cfu per ml of composition comprising milk, more preferably of 5.10⁴-1.10⁸ cfu per ml of composition comprising milk, and (2) an initial viable cell count of *Lactobacillus helveticus* of at least 5.10⁴ cfu per ml of composition comprising milk, preferably of between 5.10⁴-1.10⁹ cfu per ml of composition comprising milk, and (3) an initial viable cell count of *Lactobacillus delbrueckii* subsp. *bulgaricus* of at least 1.10³ cfu per ml of composition comprising milk, preferably 1.10⁴-1.10⁸ cfu per ml of composition comprising milk.

Additionally or alternatively hereto, prior to culturing, the composition comprising milk is preferably inoculated with the starter culture to provide a total initial viable cell count density of *Streptococcus thermophilus* and of *Lactobacillus subsp.* of at most 5.10⁷ cfu per ml of composition comprising milk, preferably of at most 1.10⁷ cfu per ml of composition comprising milk. The presence of *Lactobacillus helveticus* as an adjunct culture allows for conveniently low dosages of the starter culture whilst still providing sufficient acidification activity thereof.

For good acidification a minimum total initial viable cell count density of *Streptococcus thermophilus* and of *Lactobacillus subsp.* of preferably at least 1.10⁴ cfu per ml of composition comprising milk, more preferably of at least 5.10⁴ cfu per ml of dairy base, most preferably at least 1.10⁵ cfu per ml of composition comprising milk is recommended.

The starter culture is provided as a frozen starter culture comprising
a. bacteria of *Streptococcus thermophilus* at a total viable cell count density of at least 1.10⁸ cfu/g, preferably of at least 1.10⁹ cfu/g;
b. bacteria of *Lactobacillus delbrueckii* subsp. *bulgaricus* at a total viable cell count density of at least 1.10⁷ cfu/g, preferably at least 1.10⁸ cfu/g; and
c. bacteria of *Lactobacillus helveticus* at a total viable cell count density of least 1.10⁸ cfu/g, preferably of at least 1.10⁹ cfu/g,
**and wherein the frozen starter culture comprises less than 1.10⁶ cfu/g Lactobacillus casei.**

Herein, the expression cfu/g means "colony forming units per gram of starter culture".

In one embodiment it is preferred that the starter culture is provided as a frozen starter culture which comprises
a. frozen pellets comprising bacteria of *Streptococcus thermophilus;*
b. frozen pellets comprising bacteria of *Lactobacillus delbrueckii* subsp. *bulgaricus;* and
c. frozen pellets comprising bacteria of *Lactobacillus helveticus.*

In one embodiment, the majority of the frozen pellets, when analysed individually, preferably comprises no mixture of *Streptococcus thermophilus* and of *Lactobacillus* subsp. *bulgaricus* in a viable cell count ratio of between 1:2 - 2: 1. In one embodiment it is preferred that the majority of the frozen pellets, when analysed individually, further comprises no mixture of *Streptococcus thermophilus* and of *Lactobacillus helveticus* in a viable cell count ratio of between 1:2 - 2:1. Such a culture is conveniently obtained by mixing different cultures, preferably in frozen pellet form, each comprising a strain of *Streptococcus thermophilus,* of *Lactobacillus delbrueckii* subsp. *bulgaricus* and of *Lactobacillus helveticus,* respectively. For example, such a culture comprises at least three different types of pellets, wherein type 1 comprises only *Streptococcus thermophilus,* type 2 comprises only *Lactobacillus delbrueckii* subsp. *bulgaricus* and type 3 comprises only *Lactobacillus helveticus.* Yoghurt starter cultures comprising frozen pellets of type 1 mixed with pellets of type 2 are commercially available, for example as Y 104, ex CSK Food Enrichment BV, The Netherlands. A starter culture comprising frozen pellets of *Lactobacillus helveticus* is commercially available, for example as L100, CSK Food Enrichment BV, The Netherlands.

In other words, in one embodiment, the majority of the frozen pellets, when analysed individually, preferably comprises a mixture of *Streptococcus thermophilus* and of *Lactobacillus* subsp. *bulgaricus* in a viable cell count ratio of smaller than 1:2 or greater than 2:1. In one embodiment it is preferred that the majority of the frozen pellets, when analysed individually, further comprises a mixture of *Streptococcus thermophilus* and of *Lactobacillus helveticus* in a viable cell count ratio of smaller than 1:2 or greater than 2:1.

In another embodiment, at least 10% of the frozen pellets, when analysed individually, preferably comprises a mixture of *Streptococcus thermophilus* and of *Lactobacillus delbrueckii* subsp. *bulgaricus* in a viable cell count ratio of greater than 1:2 or smaller then 2:1. Such a culture is conveniently obtained by mixing at least 10% of a traditional yoghurt culture with a culture of *Lactobacillus helveticus,* each in frozen pellet form. A traditional yoghurt culture is a yoghurt culture which is obtainable by a method comprising co-culturing of strains of *Streptococcus thermophilus* and *Lactobacillus delbrueckii* subsp. *bulgaricus.* Examples of traditional yoghurt cultures include Y200 and Y700, each commercially available from CSK Food Enrichment BV, The Netherlands.

The composition comprising milk preferably comprises a milk solids non fat content of at least 25%, more preferably of at least 30%, yet more preferably of at least 33%. In practising the method according to the invention, assuming a certain fixed capacity of the fermentation equipment, increasing the high milk solids non fat content of the composition comprising milk allows a more efficient use of the fermentation equipment.

The composition comprising milk preferably comprises a milk solids non fat content of less than 60%, more preferably of less than 50%, most preferably of less than 45% or even of less than 40%. In order to process the fermented dairy base further, e.g. for packaging or drying purposes, it is preferred that the fermented dairy base has a sufficiently low viscosity. Thereto the milk solids non fat content of the dairy base is preferably not excessive.

In one embodiment, the composition comprising milk preferably comprises a milk solids non fat content of at least 25%, more preferably of at least 28% and of at most 40%, more preferably of at most 35%, more preferably from 28-32%.

The composition comprising milk preferably comprises water in an amount of 40-77%, more preferably in an am and wherein the composition comprising milk comprises fat in an amount of 0-15%, preferably in an amount of 0.1-10%.

Preferably the composition comprising milk comprises less than 5 wt.% sucrose, preferably less than 3 wt.%, more preferably less than 1 wt.%, based on dry weight and/or less than 5 wt.% glucose, preferably less than 3 wt.%, more preferably less than 1 wt.%, based on dry weight. Preferably the composition comprising milk comprises less than 5 wt.% maltodextrin, preferably less than 3 wt.%, more preferably less than 1 wt.%, based on dry weight. In a preferred embodiment, the composition comprising milk comprises less than 5 wt.% mono- and disaccharides other than lactose, preferably less than 3 wt.%, more preferably less than 1 wt.% mono- and disaccharides other than lactose based on dry weight.

Very good results in terms of acidification properties were obtained when the composition comprising milk was cultured at a temperature of between 37-50 °C, and even better at a temperature of between 37-45 °C, and very advantageous at a temperature of between 39-45 °C, or even 40-45 °C, and even more advantageous at a temperature form 40-43 °C, such as at around 42-44 °C. Thus in one embodiment according to the present method for producing a fermented dairy base, the composition comprising milk is cultured at a temperature of between 37-50 °C, more preferably at a temperature of between 37-45 °C, more preferably at a temperature of between 39-45 °C, even more preferably at a temperature of between 39-44 °C, even more preferably at a temperature of 40-45 °C, and even more preferably at a temperature from 40-43 °C, or at a temperature such as at around 42-44 °C.

### Preferred embodiments of the fermented dairy base and of dried products and methods for producing said products.

The fermented dairy base is preferably obtainable according to any one of the preferred embodiments of the method as defined above. The method for producing a powder preferably comprises drying a fermented dairy base which is obtainable according to any one of the preferred embodiments of the method. The powder is preferably obtainable by drying the fermented dairy base which is obtainable according to any one of the preferred embodiments of the method. Drying can be performed according to conventional drying techniques know the skilled person. In a preferred embodiment drying is performed by spray-drying. The powder is preferably indicated as a yoghurt powder.

Preferably the dried product, more preferably the powder, according to the invention comprises less than 5 wt.% sucrose, preferably less than 3 wt.%, more preferably less than 1 wt.%, based on dry weight and/or less than 5 wt.% glucose, preferably less than 3 wt.%, more preferably less than 1 wt.%, based on dry weight. Preferably the dried product, more preferably the powder, according to the invention comprises less than 5 wt.% maltodextrin, preferably less than 3 wt.%, more preferably less than 1 wt.%, based on dry weight. In a preferred embodiment, the dried product, more preferably the powder, comprises less than 5 wt.% mono- and disaccharides other than lactose, preferably less than 3 wt.%, more preferably less than 1 wt.% mono- and disaccharides other than lactose, based on dry weight.

### Preferred embodiments of the starter culture

In a preferred embodiment, the frozen starter culture according to the invention comprises less than 1.10⁶ cfu/g, preferably less than 10³ cfu/g, *Propionibacteria.* Also in a preferred embodiment, the frozen starter culture according to the invention comprises less than 1.10⁶ cfu/g, preferably less than 10³ cfu/g, *Lactococcus* spp. Also in a preferred embodiment, the frozen starter culture according to the invention comprises less than 1.10⁶ cfu/g, preferably less than 10³ cfu/g, *Leuconostoc* spp. Also in a preferred embodiment, the frozen starter culture according to the invention comprises preferably less than 10³ cfu/g, *Lactobacillus casei.*

In a preferred embodiment, the starter culture is a frozen starter culture which comprises
a. frozen pellets comprising bacteria of *Streptococcus thermophilus;*
b. frozen pellets comprising bacteria of *Lactobacillus delbrueckii* subsp. *bulgaricus;* and
c. frozen pellets comprising bacteria of *Lactobacillus helveticus.*

In a preferred embodiment, the frozen starter culture according to the invention comprises less than 1.10⁶ cfu/g, preferably less than 10³ cfu/g, *Propionibacteria.* Also in a preferred embodiment, the frozen starter culture according to the invention comprises less than 1.10⁶ cfu/g, preferably less than 10³ cfu/g, *Lactococcus* spp. Also in a preferred embodiment, the frozen starter culture according to the invention comprises less than 1.10⁶ cfu/g, preferably less than 10³ cfu/g, *Leuconostoc* spp. Also in a preferred embodiment, the frozen starter culture according to the invention comprises preferably less than 10³ cfu/g, *Lactobacillus casei.*

In a preferred embodiment, the starter culture is obtained by mixing a first type of pellets comprising bacteria of *Streptococcus thermophilus* with a second type of pellets comprising bacteria of *Lactobacillus helveticus.* The pellets comprising bacteria of *Streptococcus thermophilus* may further comprise bacteria of *Lactobacillus delbrueckii* subsp. *bulgaricus;* additionally or alternatively, in one embodiment a further type of pellets comprising bacteria of *Lactobacillus delbrueckii* subsp. *bulgaricus* is admixed.

Accordingly as explained above the majority of the frozen pellets, when analysed individually, preferably comprise no mixture of *Streptococcus thermophilus* and of *Lactobacillus delbrueckii* subsp. *bulgaricus* in a viable cell count ratio of between 1:2 - 2:1. It is especially preferred that the majority of the frozen pellets, when analysed individually, further comprise no mixture of *Streptococcus thermophilus* and of *Lactobacillus helveticus* in a viable cell count ratio of between 1:2 - 2:1. In one embodiment, when analysed individually, the frozen pellets preferably comprise a mixture of *Streptococcus thermophilus* and of *Lactobacillus delbrueckii* subsp. *bulgaricus* in a viable cell count ratio of smaller than 1:2 or greater than 2:1. In one embodiment it is preferred that the frozen pellets, when analysed individually, further comprise a mixture of *Streptococcus thermophilus* and of *Lactobacillus helveticus* in a viable cell count ratio of smaller than 1:2 or greater than 2:1.

In a preferred embodiment, the frozen starter culture comprises a cryoprotectant or cryoprotective agent. The term cryoprotectant is known in the art and denotes a substance that is able to improve the storage stability of the frozen starter culture. In a prefeered embodiment the cryoprotectant is a carbohydrate. Preferably the cryoprotectant is a carbohydrate selected from monosaccharides, more preferably selected from ribose, xylose, fructose, mannose, sorbose and glucose, disaacharides, more preferably selected from sucrose, trehalose, melibiose and lactulose, oliogosacchrdies, more preferbly selected form oligofructoses, polysacchrides, more preferably selected fom maltodextrins, xanthan gum, pectin, alginate micorcrystaline cellulose, dextrans and poly-ethylene glycol, and sugar alcohols, more preferably selected from sorbitol and mannitol, and or mixtures thereof. More preferably the cryoprotectant is selected form threhalose and sucrose and even more preferably the cryoprotectant is sucrose. Preferably the cryoprotectant is present in the frozen starter culture in an amount from 1 to 13 wt.% based on weight of the frozen starter culture.

### Examples

### Materials

Skimmed milk was concentrated by a treatment involving evaporative water removal to obtain a product containing 36% milk solids. This product was diluted with water to obtain a first milk-based composition, i.c. composition comprising milk, containing 26% milk solids ("milk base 1"). A second milk-based composition, i.c. composition comprising milk ("milk base 2") was provided by diluting milk base 1 with water to obtain a milk solids non fat content of 15%. Another milk-based composition, i.c. composition comprising milk ("milk base A") was provided by diluting the skimmed milk concentrate of 36% milk solids with water to obtain a milk solids non fat content of 30%. A further milk-based composition, i.c. composition comprising milk ("milk base B") was provided by diluting the skimmed milk concentrate of 36% milk solids with water to obtain a milk solids non fat content of 34%. The milk bases were pasteurized by subjecting them to heating at 85 deg.C for 5 minutes.

The frozen concentrated cultures Y700, L600 and L100 are commercially obtainable from CSK Food Enrichment BV, The Netherlands. Y700, L600 and L100 are commercially provided in the form of frozen pellets.

Y700 is a frozen yoghurt starter culture in pellet form comprising bacteria of *Streptococcus thermophilus* and *Lactobacillus bulgaricus.* The batch of Y700 used in the present example comprises *Streptococcus thermophilus* at a total viable cell count density of 7.4E9 cfu/g and *Lactobacillus delbrueckii* subsp. *bulgaricus* at a viable cell count density of 1.7E9 cfu/g. *S. thermophilus* and *L. bulgaricus* were enumerated on STA plates.

L100 is a frozen culture in pellet form comprising bacteria of *Lactobacillus helveticus.* The batch of L100 used in the present example comprises *Lactobacillus helveticus* at a viable cell count density of 4.3E10 cfu/g of frozen culture.

L600 is a frozen culture in pellet form comprising bacteria of *Lactobacillus helveticus.* The batch of L600 used in the present example comprises *Lactobacillus helveticus* at a viable cell count density of 2.3E10 cfu/g of frozen culture.

### Methods

In a first set of experiments, yoghurt-type fermented dairy products ("fermented dairy bases" within the context of the present invention) were prepared by inoculating milk base 1 and 2, respectively, with a starter culture comprising Y700 alone or in a mixture with L100. The inoculated milk bases thus produced were cultured at a temperature of 40 deg.C for 17 hours and the pH was monitored over time. The initial pH of the milk bases was 6.45 +/- 0.10. The time for reaching a pH value of 4.7 was determined.

In a second set of experiments, yoghurt-type fermented dairy products ("fermented dairy bases" within the context of the present invention) were prepared by inoculating milk bases A and B, respectively, with a starter culture comprising Y700 alone or in a mixture with L600. The inoculated milk bases thus produced were cultured at temperatures of 40 deg.C and 43 deg.C for 24 hours and the pH was monitored over time. The initial pH of the milk bases was 6.45 +/- 0.10. The time needed for reaching pH values of 4.8 and 4.4, respectively, was determined.

### Results.

The results for the first set of experiments are summarised in Table 1.

**Table 1**

| | | **Time to reach a pH of 4.7** | |
|---|---|---|---|
| **Starter culture** | **Dosage** (amount of starter culture / volume of milk base) | Milk base 1 (26% MSNF) | Milk base 2 (15% MSNF) |
| Y700 | 500g / 5000 litres | >> 17h* | 5h |
| Y700 mixed with L100 in a weight ratio of 70:30 | 500g / 5000 litres | 11h | 5h |
| *) Acidification curve is almost flat | | | |

Surprisingly, the added strains of *Lactobacillus helveticus* have only little (if any) effect on the time needed to lower the pH of the milk base with the lowest milk solids non fat content of 15% from a value of 6.45 initially to a value of pH 4.7. By contrast, the impact of the added strain of *Lactobacillus helveticus* in the acidification rate of the more concentrated milk base having 26% milk solids non fat content is very significant.

It has been found that a conventional yoghurt culture such as Y700 at the indicated dosage is capable to achieve an acceptable acidification rate (pH 4.7 within at most 12 hours) in a milk base having a milk solids content of up to 20-22%. If the milk solids non fat content is higher than 22%, acidification rate falls short and an added strain of *Lactobacillus helveticus* is needed to significantly reduce the time needed to lower the pH down to a value of 4.7.

The results for the second set of experiments are summarised in Tables 2A and 2B. The dosage was 500 g of starter culture per 5000 litres of milk base.

**Table 2A**

| | | **Time to reach a pH of 4.8** | | |
|---|---|---|---|---|
| **Starter culture** | Milk base B (34% MSNF), cultured at 43 deg.C | Milk base B (34% MSNF), cultured at 40 deg. C | | Milk base A (30% MSNF), cultured at 40 deg.C |
| Y700 | >24h *) | >24h *) | | >24h *) |
| Y700 mixed with L600 in a weight ratio of 70:30 | 12h30m | 14h15m | | 08h45m |
| *) Acidification curve is almost flat | | | | |

**Table 2B**

| | | **Time to reach a pH of 4.4** | | |
|---|---|---|---|---|
| **Starter culture** | Milk base B (34% MSNF), cultured at 43 deg.C | Milk base B (34% MSNF), cultured at 40 deg. C | | Milk base A (30% MSNF), cultured at 40 deg.C |
| Y700 | >24h *) | >24h *) | | >24h *) |
| Y700 mixed with L600 in a weight ratio of 70:30 | 14h35m | 16h30m | | IIh30m |
| *) Acidification curve is almost flat | | | | |

Thus, also in this example it is demonstrated that an added strain of *Lactobacillus helveticus* (in this example L600) has significant influence in reducing the acidification rate of a yoghurt starter culture in fermenting highly concentrated milk bases having 30% or 34% milk solids non fat content, as compared to experiments involving the yoghurt culture without the added strain of *Lactobacillus helveticus.*

## Claims

1. A method for producing a fermented dairy base, comprising culturing a composition comprising milk, which comprises a milk solids non fat content of at least 23%, in the presence of a starter culture comprising a strain of *Streptococcus thermophilus,* a strain of *Lactobacillus delbrueckii* subsp. *bulgaricus* and a strain of *Lactobacillus helveticus,* wherein prior to culturing, the composition comprising milk is inoculated with the starter culture to provide an initial viable cell count of *Lactobacillus helveticus* of at least 5.10⁴ cfu per ml of composition comprising milk, of *Streptococus thermophilus* of at least 1.10⁴ cfu per ml of composition comprising milk and of *Lactobacillus delbrueckii* subsp. *bulgaricus* of at least 1.10³ cfu per ml of composition comprising milk and wherein the starter culture is provided as a frozen starter culture and the frozen starter culture comprises
a. bacteria of *Streptococcus thermophilus* at a total viable cell count density of at least 1.10⁸ cfu/g of frozen starter culture, preferably of at least 1.10⁹ cfu/g;
b. bacteria of *Lactobacillus delbrueckii* subsp. *bulgaricus* at a total viable cell count density of at least 1.10⁷ cfu/g of frozen starter culture, preferably at least 1.10⁸ cfu/g; and
c. bacteria of *Lactobacillus helveticus* at a total viable cell count density of least 1.10⁸ cfu/g of frozen starter culture, preferably of at least 1.10⁹ cfu/g
**and wherein the frozen starter culture comprises less than 1.10⁶ cfu/g Lactobacillus casei.**

2. The method according to claim 1, wherein prior to culturing, the composition comprising milk is inoculated with the starter culture to provide an initial viable cell count of *Streptococus thermophilus* of 5.10⁴-1.10⁸ cfu per ml of composition comprising milk.

3. The method according to any one of the preceding claims, wherein prior to culturing, the composition comprising milk is inoculated with the starter culture to provide an initial viable cell count of *Lactobacillus helveticus* of between 5.10⁴-1.10⁹ cfu per ml of composition comprising milk.

4. The method according to any one of the preceding claims, wherein prior to culturing, the composition comprising milk is inoculated with the starter culture to provide an initial viable cell count of *Lactobacillus delbrueckii* subsp. *bulgaricus* of 1.10⁴-1.10⁸ cfu per ml of composition comprising milk.

5. The method according to any one of the preceding claims, wherein the starter culture is defined in any one of claims 10-12.

6. The method according to any one of the preceding claims, wherein the composition comprising milk comprises a milk solids non fat content of at least 25%, more preferably of at least 30%, yet more preferably of at least 33%.

7. The method according to any one of the preceding claims, wherein the composition comprising milk comprises water in an amount of 40-77% and wherein the composition comprising milk comprises fat in an amount of 0-15%, preferably in an amount of 0.1-10%.

8. The method according to any one of the preceding claims, wherein prior to culturing, the composition comprising milk is inoculated with the starter culture to provide a total initial viable cell count density of *Streptococcus thermophilus* and of *Lactobacillus* subsp. of at most 5.10⁷ cfu per ml of composition comprising milk, preferably of at most 1.10⁷ cfu per ml of composition comprising milk.

9. A frozen starter culture for preparing a fermented dairy base, the starter culture comprising
a bacteria of *Streptococcus thermophilus* at a total viable cell count density of at least 1.10⁸ cfu/g of frozen starter culture, preferably of at least 1.10⁹ cfu/g;
b bacteria of *Lactobacillus delbrueckii* subsp. *bulgaricus* at a total viable cell count density of at least 1.10⁷ cfu/g of frozen starter culture, preferably at least 1.10⁸ cfu/g; and
c bacteria of *Lactobacillus helveticus* at a total viable cell count density of least 1.10⁸ cfu/g of frozen starter culture, preferably of at least 1.10⁹ cfu/g
**and wherein the frozen starter culture comprises less than 1.10⁶ cfu/g Lactobacillus casei.**

10. The frozen starter culture according to claim 9, wherein, the starter culture comprises
a. frozen pellets comprising bacteria of *Streptococcus thermophilus;*
b. frozen pellets comprising bacteria of *Lactobacillus delbrueckii* subsp. *bulgaricus;* and
c. frozen pellets comprising bacteria of *Lactobacillus helveticus.*

11. The frozen starter culture according to claim 10 wherein the majority of the frozen pellets, when analysed individually, comprises no mixture of *Streptococcus thermophilus* and of *Lactobacillus* subsp. *bulgaricus* in a viable cell count ratio of between 1:2 - 2:1.

12. The frozen starter culture according to any one of claims 10-11 wherein the majority of the frozen pellets, when analysed individually, comprises no mixture of *Streptococcus thermophilus* and of *Lactobacillus helveticus* in a viable cell count ratio of between 1:2-2:1.

## Patentansprüche

1. Verfahren zur Herstellung einer fermentierten Milchbasis, umfassend das Kultivieren einer Zusammensetzung, umfassend Milch, die einen Milch-Nichtfett-Feststoffgehalt von mindestens 23% umfasst, in Anwesenheit einer Starterkultur, umfassend einen Stamm von Streptococcus thermophilus, einen Stamm von Lactobacillus delbrueckii subsp. bulgaricus und einen Stamm von Lactobacillus helveticus, wobei vor dem Kultivieren die Milch umfassende Zusammensetzung mit der Starterkultur angeimpft wird, um eine anfängliche Anzahl lebensfähiger Zellen von Lactobacillus helveticus von mindestens 5.10⁴ KBE pro ml Zusammensetzung, die Milch umfasst, von Streptococus thermophilus von mindestens 1.10⁴ KBE pro ml Zusammensetzung, die Milch umfasst, und von Lactobacillus delbrueckii subsp. bulgaricus von mindestens 1.10³ KBE pro ml Zusammensetzung, die Milch umfasst, bereitzustellen und wobei die Starterkultur als eine gefrorene Starterkultur bereitgestellt wird und die gefrorene Starterkultur umfasst
a. Bakterien von Streptococcus thermophilus mit einer Gesamtzahldichte der lebensfähigen Zellen von mindestens 1.10⁸ KBE/g gefrorener Starterkultur, bevorzugt mindestens 1.10⁹ KBE/g;
b. Bakterien von Lactobacillus delbrueckii subsp. bulgaricus mit einer Gesamtzahldichte der lebensfähigen Zellen von mindestens 1.10⁷ KBE/g gefrorener Starterkultur, bevorzugt mindestens 1.10⁸ KBE/g; und
c. Bakterien von Lactobacillus helveticus mit einer Gesamtzahldichte lebensfähiger Zellen von mindestens 1.10⁸ KBE/g gefrorener Starterkultur, bevorzugt mindestens 1.10⁹ KBE/g,
und wobei die gefrorene Starterkultur weniger als 1.10⁶ KBE/g Lactobacillus casei umfasst.

2. Verfahren nach Anspruch 1, wobei vor dem Kultivieren die Milch umfassende Zusammensetzung mit der Starterkultur angeimpft wird, um eine anfängliche Anzahl lebensfähiger Zellen von Streptococus thermophilus von 5.10⁴ - 1.10⁸ KBE pro ml Zusammensetzung, die Milch umfasst, bereitzustellen.

3. Verfahren nach irgendeinem der vorhergehenden Ansprüche, wobei vor dem Kultivieren die Milch umfassende Zusammensetzung mit der Starterkultur angeimpft wird, um eine anfängliche Anzahl lebensfähiger Zellen von Lactobacillus helveticus zwischen 5.10⁴ - 1.10⁹ KBE pro ml Zusammensetzung, die Milch umfasst, bereitzustellen.

4. Verfahren nach irgendeinem der vorhergehenden Ansprüche, wobei vor dem Kultivieren die Milch umfassende Zusammensetzung mit der Starterkultur angeimpft wird, um eine anfängliche Anzahl lebensfähiger Zellen von Lactobacillus delbrueckii subsp. bulgaricus von 1.10⁴ - 1.10⁸ KBE pro ml Zusammensetzung, die Milch umfasst, bereitzustellen.

5. Verfahren nach irgendeinem der vorhergehenden Ansprüche, wobei die Starterkultur in irgendeinem der Ansprüche 10 - 12 definiert ist.

6. Verfahren nach irgendeinem der vorhergehenden Ansprüche, wobei die Milch umfassende Zusammensetzung einen Milch-Nichtfett-Feststoffgehalt von mindestens 25%, bevorzugter von mindestens 30%, noch bevorzugter von mindestens 33% umfasst.

7. Verfahren nach einem der vorhergehenden Ansprüche, wobei die Milch umfassende Zusammensetzung Wasser in einer Menge von 40 - 77% enthält und wobei die Milch enthaltende Zusammensetzung Fett in einer Menge von 0 - 15%, vorzugsweise in einer Menge von 0,1 -10%, enthält.

8. Verfahren nach einem der vorhergehenden Ansprüche, wobei vor dem Kultivieren die Milch umfassende Zusammensetzung mit der Starterkultur angeimpft wird, um eine anfängliche Gesamtzahldichte der lebensfähigen Zellen von Streptococcus thermophilus und von Lactobacillus subsp. von höchstens 5.10⁷ KBE pro ml Zusammensetzung, die Milch enthält, bevorzugt von höchstens 1.10⁷ KBE pro ml Zusammensetzung, die Milch enthält, bereitzustellen.

9. Gefrorene Starterkultur zur Herstellung einer fermentierten Milchbasis, wobei die Starterkultur umfasst
a. Bakterien von Streptococcus thermophilus mit einer Gesamtzahldichte der lebensfähigen Zellen von mindestens 1.10⁸ KBE/g gefrorener Starterkultur, bevorzugt mindestens 1.10⁹ KBE/g;
b. Bakterien von Lactobacillus delbrueckii subsp. bulgaricus mit einer Gesamtzahldichte der lebensfähigen Zellen von mindestens 1.10⁷ KBE/g gefrorener Starterkultur, bevorzugt mindestens 1.10⁸ KBE/g; und
c. Bakterien von Lactobacillus helveticus mit einer Gesamtzahldichte lebensfähiger Zellen von mindestens 1.10⁸ KBE/g gefrorener Starterkultur, bevorzugt mindestens 1.10⁹ KBE/g.
und wobei die gefrorene Starterkultur weniger als 1.10⁶ KBE/g Lactobacillus casei umfasst.

10. Gefrorene Starterkultur nach Anspruch 9, wobei die Starterkultur umfasst
a. gefrorene Pellets, umfassend Bakterien von Streptococcus thermophilus;
b. gefrorene Pellets, umfassend Bakterien von Lactobacillus delbrueckii subsp. bulgaricus; und
c. gefrorene Pellets, umfassend Bakterien von Lactobacillus helveticus.

11. Gefrorene Starterkultur nach Anspruch 10, wobei der Großteil der gefrorenen Pellets, wenn sie einzeln analysiert werden, keine Mischung aus Streptococcus thermophilus und von Lactobacillus subsp. bulgaricus in einem lebensfähigen Zellzahlverhältnis zwischen 1:2 - 2:1 umfasst.

12. Gefrorene Starterkultur nach irgendeinem der Ansprüche 10 - 11, wobei der Großteil der gefrorenen Pellets, wenn sie einzeln analysiert werden, keine Mischung aus Streptococcus thermophilus und Lactobacillus helveticus in einem lebensfähigen Zellzahlverhältnis zwischen 1:2 - 2:1 umfasst.

## Revendications

1. Procédé de production d'une base laitière fermentée comprenant la mise en culture d'une composition comprenant du lait, qui comprend une teneur en matières solides non grasses de lait d'au moins 23 %, en présence d'une culture inductrice comprenant une souche de *Streptococcus thermophilus,* une souche de *Lactobacillus delbrueckii* subsp. *bulgaricus* et une souche de *Lactobacillus helveticus,* dans lequel, avant la mise en culture, la composition comprenant du lait est inoculée avec la culture inductrice pour fournir une numération cellulaire initiale viable de *Lactobacillus helveticus* d'au moins 5.10⁴ ufc par ml de composition comprenant du lait, de *Streptococus thermophilus* d'au moins 1.10⁴ ufc par ml de composition comprenant du lait et de *Lactobacillus delbrueckii* subsp. *bulgaricus* d'au moins 1.10³ ufc par ml de composition comprenant du lait et dans lequel la culture inductrice est fournie sous forme d'une culture inductrice congelée et la culture inductrice congelée comprend
a) des bactéries de *Streptococcus thermophilus* à une densité de numération cellulaire totale viable d'au moins 1.10⁸ ufc/g de culture inductrice congelée, de préférence d'au moins 1.10⁹ ufc/g ;
b) des bactéries de *Lactobacillus delbrueckii* subsp. *bulgaricus* à une densité de numération cellulaire totale viable d'au moins 1.10⁷ ufc/g de culture inductrice congelée, de préférence d'au moins 1.10⁸ ufc/g ; et
c) des bactéries de *Lactobacillus helveticus* à une densité de numération cellulaire totale viable d'au moins 1.10⁸ ufc/g de culture inductrice congelée, de préférence d'au moins 1.10⁹ ufc/g
et la culture inductrice congelée comprenant moins de 1.10⁶ ufc/g de Lactobacillus casei.

2. Procédé selon la revendication 1, dans lequel, avant la mise en culture, la composition comprenant du lait est inoculée avec la culture inductrice pour fournir une numération cellulaire initiale viable de *Streptococus thermophilus* de 5.10⁴ à 1.10⁸ ufc par ml de composition comprenant du lait.

3. Procédé selon l'une quelconque des revendications précédentes, dans lequel, avant la mise en culture, la composition comprenant du lait est inoculée avec la culture inductrice pour fournir une numération cellulaire initiale viable de *Lactobacillus helveticus* de 5.10⁴ à 1.10⁹ ufc par ml de composition comprenant du lait.

4. Procédé selon l'une quelconque des revendications précédentes, dans lequel, avant la mise en culture, la composition comprenant du lait est inoculée avec la culture inductrice pour fournir une numération cellulaire initiale viable de *Lactobacillus delbrueckii* subsp. *bulgaricus* de 1.10⁴ à 1.10⁸ ufc par ml de composition comprenant du lait.

5. Procédé selon l'une quelconque des revendications précédentes, dans lequel la culture inductrice est définie dans l'une quelconque des revendications 10 à 12.

6. Procédé selon l'une quelconque des revendications précédentes, dans lequel la composition comprenant du lait comprend une teneur en matières solides non grasses de lait d'au moins 25 %, de manière plus préférée d'au moins 30 %, de manière encore plus préférée d'au moins 33 %.

7. Procédé selon l'une quelconque des revendications précédentes, dans lequel la composition comprenant du lait comprend de l'eau selon une quantité de 40 à 77 % et dans lequel la composition comprenant du lait comprend de la matière grasse selon une quantité de 0 à 15 %, de préférence selon une quantité de 0,1 à 10 %.

8. Procédé selon l'une quelconque des revendications précédentes, dans lequel, avant la mise en culture, la composition comprenant du lait est inoculée avec la culture inductrice pour fournir une densité de numération cellulaire initiale viable de *Streptococcus thermophilus* et de *Lactobacillus* subsp. d'au plus 5.10⁷ ufc par ml de composition comprenant du lait, de préférence d'au plus 1.10⁷ ufc par ml de composition comprenant du lait.

9. Culture inductrice congelée pour la préparation d'une base laitière fermentée, la culture inductrice comprenant
a) des bactéries de *Streptococcus thermophilus* à une densité de numération cellulaire totale viable d'au moins 1.10⁸ ufc/g de culture inductrice congelée, de préférence d'au moins 1.10⁹ ufc/g ;
b) des bactéries de *Lactobacillus delbrueckii* subsp. *bulgaricus* à une densité de numération cellulaire totale viable d'au moins 1.10⁷ ufc/g de culture inductrice congelée, de préférence d'au moins 1.10⁸ ufc/g ; et
c) des bactéries de *Lactobacillus helveticus* à une densité de numération cellulaire totale viable d'au moins 1.10⁸ ufc/g de culture inductrice congelée, de préférence d'au moins 1.10⁹ ufc/g
et la culture inductrice congelée comprenant moins de 1.10⁶ ufc/g de Lactobacillus casei.

10. Culture inductrice congelée selon la revendication 9, dans laquelle la culture inductrice comprend
a) des pellets congelés comprenant des bactéries de *Streptococcus thermophilus ;*
b) des pellets congelés comprenant des bactéries de *Lactobacillus delbrueckii* subsp. *bulgaricus* ; et
c) des pellets congelés comprenant des bactéries de *Lactobacillus helveticus.*

11. Culture inductrice congelée selon la revendication 10, dans laquelle la majorité des pellets congelés, lorsqu'ils sont analysés individuellement, ne comprend aucun mélange de *Streptococcus thermophilus* et de *Lactobacillus* subsp. *bulgaricus* à un rapport de numération cellulaire viable compris entre 1:2 et 2:1.

12. Culture inductrice congelée selon l'une quelconque des revendications 10 à 11, dans laquelle la majorité des pellets congelés, lorsqu'ils sont analysés individuellement, ne comprend aucun mélange de *Streptococcus thermophilus* et de *Lactobacillus helveticus* à un rapport de numération cellulaire viable compris entre 1:2 et 2:1.
